(19) Europäisches Patentamt
European Patent Office
Office européen des brevets

(11) Publication number: **0 085 996**
**B1**

(12) **EUROPEAN PATENT SPECIFICATION**

(45) Date of publication of patent specification: **04.03.87**

(51) Int. Cl.[4]: **C 07 C 45/45**, C 07 C 49/04, B 01 J 23/10

(21) Application number: **83200056.6**

(22) Date of filing: **14.01.83**

# (54) Process for the preparation of unsymmetrical aliphatic ketones.

(30) Priority: **08.02.82 GB 8203562**

(43) Date of publication of application:
**17.08.83 Bulletin 83/33**

(45) Publication of the grant of the patent:
**04.03.87 Bulletin 87/10**

(84) Designated Contracting States:
**BE DE FR GB IT NL**

(56) References cited:
**DE-A-2 737 511**
**FR-A-2 038 732**
**FR-A-2 438 643**
**GB-A-1 194 057**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Wattimena, Freddy**
**Badhuisweg 3**
**NL-1031 CM Amsterdam (NL)**

(74) Representative: **Aalbers, Onno et al**
**P.O. Box 302**
**NL-2501 CH The Hague (NL)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 085 996 B1

## Description

This invention relates to a process for the preparation of unsymmetrical aliphatic ketones by heating two different carboxylic acids having at least two aliphatic carbon atoms in the presence of a catalyst.

Ketones may be represented by the general formula $R^1—CO—R^2$, wherein $R^1$ and $R^2$ are hydrocarbyl groups. In a symmetrical ketone $R^1$ and $R^2$ are identical, whereas in an unsymmetrical ketone they are not. If $R^1$ and $R^2$ together form one biradical group, the ketone is cyclic.

Ketones may be prepared by the so-called decarboxylative condensation of two carboxylic acids (excluding formic acid) according to the following general equation:

$$R^1COOH+R^2COOH \xrightarrow[\text{catalyst}]{\text{heat}} R^1—CO—R^2+CO_2+H_2O$$

Obviously, if $R^1=R^2$, only the symmetrical ketone $R^1—CO—R^1$ will be formed, and quite high yields have been obtained using various catalysts, e.g. thoria. However, if $R^1$ and $R^2$ represent different hydrocarbyl groups, generally a mixture of three ketones, two symmetrical and one unsymmetrical, is formed. The above reaction therefore is not considered to be generally suited for the preparation of unsymmetrical ketones, due to the presence of two, sometimes difficultly removable, symmetrical ketones, and the low selectivity associated therewith.

In FR—A—2 438 643 there is described the preparation of a symmetrical aliphatic ketone, 4-heptanone, by heating n-butyric acid in the presence of a catalyst consisting of lanthanum (in the form of $La_2O_3$) on alumina.

GB—A—1 194 057 discloses a process for the preparation of an unsymmetrical aliphatic ketone, e.g. methylisopropyl ketone, by heating an aliphatic aldehyde, e.g. acetaldehyde, and an aliphatic carboxylic acid, e.g. isobutyric acid, in the presence of at least one rare-earth metal, as the oxide, on an alumina carrier.

It has been described by M. B. Turova-Polyak *et al.*, Doklady Akad. Nauk SSSR Vol. 157, No. 3 pages 643—5, July 1964, to use certain rare earth metals deposited on quartz or asbestos, in order to catalyze the above decarboxylative condensation of carboxylic acids. Apparently these catalysts are quite active in the preparation of symmetrical ketones, but the yield of unsymmetrical aliphatic ketones—when using two different carboxylic acids—is very low. One would be inclined to conclude that supported rare earth metals are not very effective in the preparation of unsymmetrical aliphatic ketones from two different aliphatic carboxylic acids.

Surprisingly it has now been found that in the decarboxylative condensation of two different aliphatic carboxylic acids the formation of symmetrical ketones can be suppressed and, simultaneously, the formation of unsymmetrical ketones can be promoted, by employing a catalyst based on gamma-alumina, and also containing rare earth metals.

Accordingly the invention relates to a process for the preparation of unsymmetrical aliphatic ketones by heating two different carboxylic acids having at least two aliphatic carbon atoms in the presence of a rare earth metal containing catalyst, characterised in that the catalyst comprises one or more rare earth metals on a gamma-alumina-based carrier.

Suitably the catalyst contains from 5 to 30% of rare earth metal based on the weight of the carrier, preferably from 10 to 28% and in particular from 17 to 26%. It has also been found that increasing the amount of rare earth metal promotes the yield of unsymmetrical ketone, at the expense of the formation of symmetrical ketones. The yield of the unsymmetrical ketone, however, becomes constant at higher rare earth metal percentages, and for this reason the range of from 17 to 26% by weight is particularly preferred as already stated. These amounts refer to the rare earth elements calculated as metal, although mostly these metals are present as compounds, e.g. as oxides, sulphides and the like.

The carrier is based on gamma-alumina and may contain minor amounts of other known carrier materials. The expression "based on" as used herein means that the carrier should consist of $\gamma Al_2O_3$ for at least 70% by weight. It has been observed, however, that the addition of CaO or $Li_2O$, in general the addition of alkali metal or alkaline earth metal oxides, decreases the selectivity towards the unsymmetrical ketone desired. The same adverse effect has also been observed with $SiO_2$-based carriers such as quartz or silica, and with $SiO_2—Al_2O_3$ mixed carriers, including asbestos. These oxides preferably are virtually absent from the present catalyst, therefore. Preferably a pure gamma-alumina is used.

In order to be able to increase the content of rare earth metal it is advantageous to use a carrier material with a large specific area. Excellent results have been obtained with the gamma-alumina having a specific area of 50 to 260 $m^2/g$. Such aluminas are commercially available, both for fluid and for fixed bed application.

The rare earth metals comprise the elements scandium, yttrium, lanthanum and the lanthanides, i.e. the elements having atomic numbers 58 to 71 inclusive. The rare earth elements often are found together and are rather difficult to separate. Conveniently, therefore, sometimes mixtures of rare earth elements in naturally occurring ratios are used, without determining which element actually is the most active catalyst.

Very satisfactory results have been obtained when as earth metal lanthanum, cerium, praeseodymium

and/or neodymium is used. A mixture of rare earth elements excluding cerium is commercially obtainable—at a reasonable price—under the name of didymium, and excellent results have been obtained herewith. Preferably therefore a mixture of (at least) lanthanum, praeseodymium and neodymium, in a naturally or commercially occurring ratio, is used as rare earth metal [please refer to Kirk-Othmer, Encyclopedia of Chemical Technology (1968), Vol. 17, p. 147, for more information on rare earth mixtures].

The rare earth metal or metals may be introduced onto the carrier by any convenient method, for example by impregnation of aqueous solutions of soluble salts of the rare earth metal(s). Preferably the rare earth metal(s) is (are) present as oxide(s). Suitably the carrier is dried and calcined after a solution of rare earth metal nitrate or acetate has been absorbed. The calcination is effected preferably at a temperature in the range from 350 to 600°C.

The two different carboxylic acids should have at least two carbon atoms, which means that acetic acid and higher homologs can be used in the preparation of ketones. Formic acid, however, can not be used since it gives rise to aldehydes, which usually decompose. The smallest unsymmetrical ketone is methyl ethyl ketone which, according to the present invention, may be prepared from acetic acid and propionic acid. Suitably two different carboxylic acids are used having not more than 30 carbon atoms each.

The carboxylic acids may be aliphatic including arylaliphatic; e.g. phenylacetic acid is included.

Excellent results are obtained when acetic acid is used as carboxylic acid. The invention thus provides in particular a process for the preparation of methyl ketones, $CH_3(CO)R$, in which R represents a hydrocarbyl group, preferably an alkyl group, by reacting acetic acid with a carboxylic acid RCOOH.

In a preferred embodiment an aliphatic acid having no or only one alpha-hydrogen atom is used as carboxylic acid. It appears that such acids give rise to relatively little homocondensation, so that still higher selectivities towards the unsymmetrical ketone are obtained. Acids having no alpha-hydrogen atom include pivalic or trimethylacetic acid, trichloroacetic acid and α,α-dimethylbutyric acid. Pivalic acid is preferably used as the acid having no alpha-hydrogen atom, since after reaction with acetic acid it yields the important chemical product pinacolone, or tertiary butyl methylketone. Examples of acids having only one alpha-hydrogen atom are isobutyric acid, α-methylbutyric acid, α-bromopropionic acid and α-phenylpropionic acid. Preferably isobutyric acid is used as the acid having only one alpha-hydrogen atom, since after reaction with acetic acid it yields the important solvent methyl isopropyl ketone.

It is also possible to use the anhydride form of carboxylic acids. For instance, passing acetic anhydride over the catalyst according to the invention results in the formation of dimethyl ketone (acetone), and passing acetic anhydride mixed with another acid would result in an unsymmetrical ketone.

According to the invention the selectivity towards unsymmetrical ketones is increased and consequently the yield of symmetrical ketones is relatively small. It has been found, however, that the catalyst according to the invention also catalyses the reaction between a symmetrical ketone and a carboxylic acid to yield an other ketone. This now makes it possible to increase the yield of unsymmetrical ketone, by recycling any undesired symmetrical ketones and converting these to the desired unsymmetrical ketone. Advantageously thus any symmetrical ketone formed is separated from the unsymmetrical ketone and subsequently recycled to the catalyst.

The molar ratio of the two different carboxylic acids may be roughly stoechiometric, i.e. about 1, but rather large excesses of one of the two may have some advantages, sometimes. If one of the two carboxylic acids happens to have a relatively low homocondensation tendency, as is the case with acids having no α-H atoms, it may be present in excess to the other acid in order to suppress any homocondensation of that acid. The already high yield of unsymmetrical ketone may thus be further increased. On the other hand it is desirable to use an excess of the cheaper acid in order to convert all of the more expensive acid into unsymmetrical ketone. Usually, acids having no or one α-H atom are relatively expensive. The molar ratio therefore, of the two carboxylic acids preferably lies between 10 and 0.1, in particular between 5 and 0.2, depending on the above considerations.

These ratio limits may be extended even further if one of the two acids, preferably the one with the higher homocondensation tendency, is slowly and gradually added to a large volume of the other acid. At the beginning of the reaction the molar ratio thus is almost zero (or infinity), at the end it may be one.

The most preferred way of reacting the two acids, however, is to feed them—premixed—over a heated catalyst. The reactants may have been preheated to effect their vaporization, e.g. to a temperature of 100 to 400°C.

The preparation, i.e. the actual decarboxylative (hetero) condensation reaction, is preferably carried out at a temperature in the range of 380 to 580°C, in particular of 425 to 525°C. Advantageously the preparation is carried out at a weight hourly space velocity of 0.1 to 5 kg carboxylic acids/kg catalyst/hour. The two carboxylic acids may be used per se, or diluted with an inert carrier gas such as nitrogen or argon. Air, however, is not inert and causes a decrease in selectivity towards the desired unsymmetrical ketone. Addition of steam has no detrimental effect on the production rate of unsymmetrical ketone, and has the extra advantage of keeping the carbon deposits on the catalyst at a low level, thus reducing the frequency of regeneration. Preferably, therefore, the preparation is carried out in the presence of steam. The molar ratio of the carboxylic acids (together) to the steam preferably lies between 0.1 and 100, in particular between 0.5 and 5.0.

The pressure used may be atmospheric pressure or slightly above.

Examples

The following Examples illustrate the invention. The identity of the products was confirmed by infra-red and nuclear magnetic resonance analysis and by gas-liquid chromatography as necessary.

All catalysts were prepared by adding a solution of rare earth metal nitrate in water to the carrier in an amount equal to its pore-volume, and removing the water in the pores by stirring the mixture at about 100—150°C. Then the catalysts were calcined in air at 500°C for 2 hours. All catalyst compositions are given in pbw of metal per 100 parts of carrier.

6 g of calcined catalyst were transferred to a micro-fluid-bed reactor. A mixture of carboxylic acids, steam and nitrogen, at atmospheric pressure, was fed through the catalyst at various temperatures (T,°C), various molar ratios and various weight hourly space velocities (WHSV, kg/kg/hour). This corresponded generally to a residence time of 1—5 seconds. The conversion of acid and the yield of ketones were measured by gas liquid chromatography and nmr analysis.

Example 1

The reaction of pivalic acid and acetic acid to yield pinacolone was tested using four catalysts based on gamma-alumina, E-type, i.e. an alumina suitable for use in fluid beds, having a specific surface area of 125 $m^2/g$ and manufactured by Ketjen. All catalysts contained 17.2 parts by weight of metal, based on 100 parts of alumina carrier. Actually the metals were present as oxides, i.e. (presumably) as $Di_2O_3$, $CeO_2$, $Nd_2O_3$, or $La_2O_3$. $Di_2O_3$ stands for didymium oxide, a rare earth metal mixture having the following composition: 45.8% $La_2O_3$, 9.5% $Pr_6O_{11}$, 32.5% $Nd_2O_3$, 5.5% $Sm_2O_3$, 3.5% $Gd_2O_3$, 0.5% $Y_2O_3$, 2.7% other rare earth oxides (all percentages are by weight). The WHSV and the conversion percentage of pivalic acid (PAC) and the yield molar percentage of pinacolone (PON) are tabulated in Table I, together with the calculated selectivity of pivalic acid towards pinacolone. The molar ratio of pivalic acid, acetic acid, steam and nitrogen amounted 1/1.4/1.4/1.1.

TABLE I

| Experiment | Catalyst | WHSV kg/kg/h PAC | T °C | Conversion PAC, % | Yield PON, %m | Selectivity PON, % |
|---|---|---|---|---|---|---|
| 1 | 17.2 Di | 0.96 | 431 | 53.0 | 48.9 | 92.3 |
| 2 | 17.2 Di | 0.96 | 415 | 33.5 | 32.7 | 97.6 |
| 3 | 17.2 Di | 0.96 | 450 | 82.0 | 74.3 | 90.6 |
| 4 | 17.2 Ce | 0.97 | 430 | 36.4 | 33.8 | 92.9 |
| 5 | 17.2 Nd | 0.97 | 435 | 59.6 | 57.8 | 97.0 |
| 6 | 17.2 La | 0.99 | 430 | 50.7 | 47.8 | 94.3 |
| 7 | 17.2 La | 0.49 | 430 | 88.4 | 76.0 | 86.0 |

It can be seen that of the metals tested, cerium is less effective than didymium, lanthanum is about equally active and neodymium is more effective than didymium in the synthesis of pinacolone.

Example 2

The reaction between pivalic acid and acetic acid according to Example 1 was carried out using a series of didymium catalysts, based on different carriers, including a silica and a silica-alumina carrier, as comparative examples. The results are shown in Table II, using the same units as in Table I, but adding the yield figures of dimethyl ketone (DMK). The molar yield to DMK refers to the conversion of acetic acid to dimethyl ketone, whereas the yield to PON refers to the conversion of pivalic acid (and acetic acid) to pinacolone. It should be noted that 1 mole of acetic acid yields 1/2 mole of DMK and that 1 mole of pivalic acid yields 1 mole of PON, assuming 100% conversion and the selectivity. In all experiments a mixture of pivalic acid/acetic acid/steam/nitrogen was fed to the reactor in a molar ratio of 1/1.4/1.4/1.1, except in experiment 14, where this ratio was 1/2/2/2.5.

TABLE II

| Experiment | Catalyst | T °C | WHSV kg/kg/h PAC | Conv. % PAC | Yield %m PON | Yield %m DMK | Sel. % PON |
|---|---|---|---|---|---|---|---|
| 8* | 19.6 $Di/SiO_2$ [1)] | 468 | 2.10 | 46.3 | 19.3 | 73.8 | 41.7 |
| 9* | 19.6 $Di/SiO_2—Al_2O_3$ [2)] | 465 | 2.48 | 100 | 0 | 64.8 | 0 |
| 10 | 17.2 $Di/Al_2O_3$ [3)] | 480 | 0.96 | 99.5 | 92.4 | 33.6 | 92.9 |
| 11 | 17.2 $Di/Al_2O_2$ [3)] | 450 | 0.96 | 82.0 | 74.3 | 46.9 | 90.6 |
| 12 | 26.2 $Di/Al_2O_3$ [4)] | 398 | 1.01 | 19.4 | 19.3 | 77.8 | 99.5 |
| 13 | 8.6 $Di/Al_2O_3$ [3)] | 462 | 0.97 | 54.2 | 50.8 | 57.3 | 93.7 |
| 14* | 8.6 $Di/Al_2O_3$ [5)] | 530 | 0.83 | 38.5 | 38.5 | 70.6 | 99.9 |

* comparative
1) silica, type F-22 ex. Ketjen, specific surface area 400 $m^2/g$.
2) silica-alumina, type LA-HPV ex Ketjen, S. A. 600$m^2/g$, containing 13%w $Al_2O_3$.
3) γ alumina, type E ex Ketjen, S. A. 125$m^2/g$.
4) γ alumina, type D ex Ketjen, S. A. 250$m^2/g$.
5) α alumina, type SAEHS-5, ex Carborundum, S. A. 1.4$m^2/g$.

Evidently the catalysts based on pure alumina are superior to those based on silica or silica-alumina: the pivalic acid conversion and/or the selectivity towards pinacolone is better, whereas the yield of dimethylketone is lower (note, however, that experiment 12 represents a non-optimized case at a too low temperature). The gamma-alumina having a specific surface area of 125$m^2/g$ gives the best results.

Example 3

Example 2 was repeated using catalysts based on alumina but containing different amounts of didymium. The results are shown in Table III, using the same abbreviations, notes and units as in Table II. In experiments 15 the molar ratio pivalic acid/acetic acid/steam/nitrogen was 1/4/4/4, in experiment 16 it was 1/2/2/2.5, and in experiments 17—19 it was 1/1.4/1.4/1.1.

TABLE III

| Experiment | Catalyst | T °C | WHSV kg/kg/h PAC | Conv. % PAC | Yield %m PON | Yield %m DMK | Sel. % PON |
|---|---|---|---|---|---|---|---|
| 15* | 5.7 $Di/Al_2O_3$ [5)] | 530 | 0.21 | 99.5 | 87.6 | 77.2 | 88.0 |
| 16* | 8.6 $Di/Al_2O_3$ [5)] | 530 | 0.33 | 60.1 | 55.5 | 65.0 | 92.3 |
| 17 | 8.6 $Di/Al_2O_3$ [3)] | 438 | 0.97 | 30.4 | 29.6 | 69.5 | 97.4 |
| 18 | 17.2 $Di/Al_2O_3$ [3)] | 431 | 0.96 | 53.0 | 48.9 | 57.1 | 92.3 |
| 19 | 26.2 $Di/Al_2O_3$ [3)] | 432 | 0.83 | 62.6 | 61.3 | 53.2 | 97.9 |

It appears that increasing the didymium concentration and/or the surface of the support resulted in a more effective consumption of acetic acid towards pinacolone (relatively less homocondensation). This is reflected in a decrease of the acetic acid/pivalic acid molar ratio.

Example 4

Experiments 17, 18 and 19 of the preceding Example were repeated at other temperatures and space velocities. It appeared that both the activity and the selectivity of the catalyst increased with increasing didymium content. After plotting the activity versus the Di-content, it was concluded that the most efficient catalyst should contain 20 parts Di per 100 parts $\gamma Al_2O_3$.

Example 5

The catalyst containing 26.2% w Di on 100% $Al_2O_3$(E) was chosen for further experiments, including recycle of symmetrical product to increase the yield of unsymmetrical ketone. It was found that feeding a mixture of pivalic acid, acetic acid, steam and nitrogen, having a molar ratio of 1/1.15/2.7/1, at a WHSV of 0.90kg · $kg^{-1}$ · $h^{-1}$ pivalic acid and a temperature of 520°C, resulted in a product stream containing 0.2 mol acetone, 0.9 mol pinacolone and 0.1 mol pivalic acid per mol of pivalic acid fed to the reactor. Recycle of the acetone formed was simulated by feeding to the reactor the above mentioned mixture, plus 0.2 mol acetone per mol of pivalic acid. This resulted, under the same conditions, in 100% conversion of pivalic acid to pinacolone, i.e. with a selectivity of 100%.

This catalyst was further tested under the above conditions for an extended period of 60 hours. No deactivation was observed.

Example 6

The catalyst of Example 5 was tested using a mixture of pivalic acid, propionic acid and steam (molar ratio: 1/1.6/3.2) at a WHSV of 0.7kg pivalic acid/kg catalyst/hour, at atmospheric pressure and at temperatures of 505 and 530°C, respectively. The products were absorbed in $CH_3OH$ and analyzed by gas liquid chromatography. The resulting weight percentages are shown in Table IV.

TABLE IV

| Product | 505°C | 530°C |
|---|---|---|
| propionic acid | trace | — |
| pivalic acid | 27.4 | 3.0 |
| diethyl ketone | 37.0 | 42.7 |
| t.butylethyl ketone | 31.2 | 36.0 |
| unknown | 3.3 | 11.5 |

Notwithstanding the fact that propionic acid has two alpha hydrogen atoms, and a rather high homocondensation tendency therefore, a sizeable amount of the hetero-condensate is formed, which amount could even be increased by recycling the diethyl ketone formed.

Example 7

Example 6 was repeated using a mixture of isobutyric acid, acetic acid and steam (molar ratio: 1/1.5/2.5) at a WHSV of 0.77kg isobutyric acid/kg catalyst/hour and temperatures of 390, 425, 450 and 485°C, respectively. The resulting weight percentages are given in Table V.

TABLE V

| Product | 390°C | 425°C | 450°C | 485°C |
|---|---|---|---|---|
| acetic | — | — | — | — |
| isobutyric acid | 30.4 | — | — | — |
| dimethyl ketone | 22.7 | 13.0 | 11.8 | 11.1 |
| methylisopropyl ketone | 46.9 | 83.9 | 83.3 | 80.6 |
| diisopropyl ketone | — | 1 | 4.9 | 6.5 |

A sizeable amount of the important solvent methylisopropyl ketone is formed. Recycle of symmetrical products would even increase the yield of unsymmetrical product.

**Claims**

1. Process for the preparation of unsymmetrical aliphatic ketones by heating two different carboxylic acids having at least two aliphatic carbon atoms in the presence of a rare earth metal containing catalyst, characterized in that the catalyst comprises one or more rare earth metals on a gamma-alumina-based carrier.

2. A process as claimed in claim 1, characterized in that the catalyst contains from 5 to 30% of rare earth metal based on the weight of the carrier.

3. A process as claimed in claim 1 or 2, characterized in that the gamma-alumina has a specific area of 50 to 260$m^2$/g.

4. A process as claimed in any one of claims 1 to 3, characterized in that as rare earth metal lanthanum, cerium, praeseodymium and/or neodymium is used.

5. A process as claimed in any one of claims 1 to 4, characterized in that the rare earth metal(s) is (are) present as oxide(s).

6. A process as claimed in any one of claims 1 to 5, characterized in that as carboxylic acid acetic acid is used.

7. A process as claimed in any one of claims 1 to 6, characterized in that as carboxylic acid an aliphatic acid having no or only one alpha-hydrogen atom is used.

8. A process as claimed in claim 7, characterized in that pivalic acid issued as the acid having no alpha-hydrogen atom.

9. A process as claimed in claim 7, characterized in that isobutyric acid is used as the acid having only one alpha-hydrogen atom.

10. A process as claimed in any one of claims 1 to 9, characterized in that any symmetrical ketone formed is separated from the unsymmetrical ketone and subsequently recycled to the catalyst.

11. A process as claimed in any one of claims 1 to 10, characterized in that the preparation is carried out at a weight hourly space velocity of 0.1 to 5kg carboxylic acids/kg catalyst/hour, a temperature of 380 to 580°C and a molar ratio of the two different carboxylic acids between 10 and 0.1.

**Patentansprüche**

1. Verfahren zur Herstellung von unsymmetrischen aliphatischen Ketonen durch Erhitzen von zwei verschiedenen Carbonsäuren mit mindestens zwei aliphatischen Kohlenstoffatomen in Gegenwart eines ein seltenes Erdmetall enthaltenden Katalysators, dadurch gekennzeichnet, daß der Katalysator ein oder mehrere seltene Erdmetalle auf einem Träger auf Basis von γ-Tonerde umfaßt.

2. Verfahren nach Anspruch 1, dadurch gekennzeichnet, daß der Katalysator 5 bis 30% seltenes Erdmetall, bezogen auf das Gewicht des Trägers, enthält.

3. Verfahren nach Anspruch 1 oder 2, dadurch gekennzeichnet, daß die γ-Tonerde eine spezifischen Oberfläche von 50 bis 260 $m^2$/g besitzt.

4. Verfahren nach einem der Ansprüche 1 bis 3, dadurch gekennzeichnet, daß als seltenes Erdmetall Lanthan, Cer, Praseodym und/oder Neodym verwendet wird.

5. Verfahren nach einem der Ansprüche 1 bis 4, dadurch gekennzeichnet, daß das (die) seltene(n) Erdmetall(e) als Oxid(e) vorliegt (vorliegen).

6. Verfahren nach einem der Ansprüche 1 bis 5, dadurch gekennzeichnet, daß als Carbonsäure Essigsäure verwendet wird.

7. Verfahren nach einem der Ansprüche 1 bis 6, dadurch gekennzeichnet, daß als Carbonsäure eine aliphatische Säure verwendet wird, die kein oder nur ein α-Wasserstoffatom besitzt.

8. Verfahren nach Anspruch 7, dadurch gekennzeichnet, daß Pivalinsäure als Säure, die kein α-Wasserstoffatom besitzt, verwendet wird.

9. Verfahren nach Anspruch 7, dadurch gekennzeichnet, daß Isobuttersäure als Säure mit nur einem α-Wasserstoffatom verwendet wird.

10. Verfahren nach einem der Ansprüche 1 bis 9, dadurch gekennzeichnet, daß ein etwa entstandenes symmetrisches Keton von dem unsymmetrischen Keton abgetrennt und der Katalysator anschließend zurückgeführt wird.

11. Verfahren nach einem der Ansprüche 1 bis 10, dadurch gekennzeichnet, daß die Herstellung mit einer Raumgeschwindigkeit von 0,1 bis 5 kg Carbonsäure/kg Katalysator je Stunde bei einer Temperatur von 380 bis 580°C und einem Molverhältnis der beiden verschiedenen Carbonsäuren zwischen 10 und 0,1 durchgeführt wird.

**Revendications**

1. Procédé de préparation de cétones aliphatiques asymétriques par chauffage de deux acides carboxyliques différents comportant au moins deux atomes de carbone aliphatiques en présence d'un catalyseur contenant un métal de terre rare, caractérisé en ce que le catalyseur comprend un ou plusieurs métaux de terres rares sur un support à base de gamma-alumine.

2. Procédé selon la revendication 1, caractérisé en ce que le catalyseur contient de 5 à 30% de métal de terre rare par rapport au poids du support.

3. Procédé selon la revendication 1 ou 2, caractérisé en ce que la gamme-alumine a une surface spécifique de 50 à 260 $m^2$/g.

4. Procédé selon l'une quelconque des revendications 1 à 3, caractérisé en ce que l'on utilise comme métal de terre rare le lanthane, le cérium, le praséodyme et/ou le néodyme.

5. Procédé selon l'une quelconque des revendications 1 à 4, caractérisé en ce que le ou les métaux de terres rares est (sont) présent(s) sous forme d'oxyde(s).

6. Procédé selon l'une quelconque des revendications 1 à 5, caractérisé en ce que l'on utilise l'acide acétique comme acide carboxylique.

7. Procédé selon l'une quelconque des revendications 1 à 6, caractérisé en ce que l'on utilise comme acide carboxylique un acide aliphatique ne possédant aucun, ou ne possédant qu'un, atome d'hydrogène en alpha.

8. Procédé selon la revendication 7, caractérisé en ce que l'on utilise l'acide pivalique comme acide ne possédant aucun atome d'hydrogène en alpha.

9. Procédé selon la revendication 7, caractérisé en ce que l'on utilise l'acide isobutyrique comme acide ne possédant qu'un atome d'hydrogène en alpha.

10. Procédé selon l'une quelconque des revendications 1 à 9, caractérisé en ce que toute cétone symétrique formée est séparée de la cétone asymétrique et ensuite recyclée vers le catalyseur.

11. Procédé selon l'une quelconque des revendications 1 à 10, caractérisé en ce que la préparation s'effectue à une vitesse spatiale horaire en poids de 0,1 à 5 kg d'acides carboxyliques/kg de catalyseur/heure, à une température de 380 à 580°C et en un rapport molaire des deux acides carboxyliques différents compris entre 10 et 0,1.